(19) Europäisches Patentamt
European Patent Office
Office européen des brevets

(11) Numéro de publication : **0 105 785 B1**

(12) # FASCICULE DE BREVET EUROPÉEN

(45) Date de publication du fascicule du brevet : **02.03.88**

(51) Int. Cl.⁴ : **C 07 J 51/00**, G 01 N 33/74

(21) Numéro de dépôt : **83401848.3**

(22) Date de dépôt : **22.09.83**

(54) **Complexes organométalliques d'oestrogènes et leur application au dosage des récepteurs hormonaux.**

(30) Priorité : **23.09.82 FR 8216024**

(43) Date de publication de la demande : **18.04.84 Bulletin 84/16**

(45) Mention de la délivrance du brevet : **02.03.88 Bulletin 88/09**

(84) Etats contractants désignés : **AT BE CH DE FR GB IT LI LU NL SE**

(56) Documents cités :
**FR-A- 2 353 854**
**JOURNAL OF INORGANIC AND NUCLEAR CHEMISTRY, vol. 42, 1980, pages 1659-1662, Pergamon Press, Oxford, GB G. POUSKOULELI et al.: "Reactions of group VIB metal hexacarbonyls with arene hormonal steroids"**

(73) Titulaire : **Etablissement Public dit: CENTRE NATIONAL DE LA RECHERCHE SCIENTIFIQUE (CNRS)**
**15, Quai Anatole France**
**F-75007 Paris (FR)**

(72) Inventeur : **Jaouen, Gérard**
**15, Allée du Parc de la Bièvre**
**F-94240 L'Hay-les-Roses (FR)**
Inventeur : **Vessieres, Anne**
**15, Allée du Parc de la Bièvre**
**F-94240 L'Hay-les-Roses (FR)**
Inventeur : **Top, Siden**
**38, rue Marie Roche Lisses**
**F-91000 Evry (FR)**

(74) Mandataire : **Warcoin, Jacques et al**
**Cabinet Régimbeau 26, avenue Kléber**
**F-75116 Paris (FR)**

**EP 0 105 785 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

Dans un domaine en pleine expansion comme la chimie organométallique moléculaire, les axes d'applications essentiels se sont jusqu'à présent cantonnés à des synthèses organiques stoechiométriques et catalytiques.

La présente invention propose un nouveau domaine d'application pour ces dérivés de la chimie organométallique moléculaire, il s'agit en particulier de la détection et du dosage des récepteurs hormonaux.

L'événement primaire qui initie l'action de la majorité des hormones peptidiques et stéroïdes et des médicaments consiste dans leur association avec une protéine spécifique, appelée « récepteur », localisée sur la membrane cellulaire (cas des hormones peptidiques) ou dans le cytoplasme (pour les hormones stéroïdes). Dans la mesure où les récepteurs interviennent dans l'action de la substance biochimique ou du médicament, on s'attend à ce que des changements dans la concentration du récepteur reflètent l'état de la maladie.

De nombreux systèmes illustrent la justesse d'un tel concept (J. P. Raynaud, T. Ojasoo, M. M. Bouton et D. Philibert, « Drug Design » vol. VIII, Acad. Press. (1979) p. 170-214).

Par exemple dans le cas des tumeurs humaines du sein, un cas particulier des cancers hormonodépendants ; il y a relativement peu de récepteurs œstrogéniques dans le tissu mammaire sain mais la quantité en devient importante dans environ la moitié des cancers primaires du sein. Un taux de 73 % de rémission a été obtenu pour des patients dont la tumeur contient de hautes concentrations de récepteurs œstrogéniques tandis qu'un taux de 2 % seulement de rémission a été observé en leur absence (W. L. Mc Guire, « Hormones and Cancer » vol. 14, Raven Press, (1980) p. 337-343).

Jusqu'ici, essentiellement pour des raisons de sensibilité, les méthodes de dosage des récepteurs sont basées sur des techniques radio-isotopiques en dépit de leurs désavantages bien connus (coûts élevés, limitations légales, risques pour la santé, variété limitée des isotopes utilisables, difficultés de marquage, instabilités chimique et biochimique).

Ainsi, l'article de J. Inorg. nucl. Chem., vol. 42, (1980) pp. 1659-1662 décrit des composés complexés par $Cr(CO)_3$ qui sont instables en solution et ne peuvent être utilisés à des fins d'analyse biochimique.

La présente invention se propose de remplacer les techniques des marquages radio-isotopiques des œstrogènes par un marquage à l'aide de complexes de métaux carbonyle, en utilisant une propriété unique des complexes de métaux carbonyle, à savoir l'existence d'une bande I. R. $\bar{\nu}$ CO dans la région 1 900-2 000 $cm^{-1}$, en fait juste dans la « fenêtre » laissée libre dans les protéines.

Pour ce faire, la présente invention propose de nouveaux composés utiles notamment pour la détection et le dosage des récepteurs hormonaux qui sont constitués par des complexes d'œstrogènes comportant un œstrogène ou un dérivé d'œstrogène complexé par un composé organométallique possédant au moins un ligand carbonyle libre, ledit complexe d'œstrogène comportant au moins un radical hydroxy libre et aucun radical hydroxy phénolique libre en $\alpha$ du site de fixation du composé organométallique, les composés organométalliques étant des composés des métaux des groupes VIa, VIIa et VIII de la Classification Périodique.

Dans la définition des composés précédents, il faut remarquer :

— que l'existence d'un radical hydroxy libre est indispensable afin que le complexe obtenu présente un taux de reconnaissance raisonnable du récepteur spécifique ;

— que l'absence de radical hydroxy phénolique en $\alpha$, c'est-à-dire sur le carbone adjacent, du carbone où se trouve placé le composé organométallique est imposée pour des raisons de stabilité et, enfin

— que la présence d'un ligand carbonyle libre sur le composé organométallique est indispensable puisque c'est précisément cette fonction qui permettra de détecter le complexe.

Parmi les œstrogènes et les dérivés d'œstrogènes utilisables dans le cadre de la présente invention, il convient de citer notamment l'œstradiol, l'œstrone, la 16$\alpha$-hydroxy-œstrone, l'œstriol, l'éthynyloestradiol qui sont des stéroïdes hormonaux, ainsi que des œstrogènes non stéroïdiens, en particulier les dérivés du diphényléthane et du stilbène, tels que le diéthylstilbœstrol et l'hexœstrol.

Comme cela a été indiqué précédemment, il est possible d'utiliser des dérivés d'œstrogènes, en particulier il est possible, si cela est nécessaire, de protéger certaines des fonctions hydroxy afin d'améliorer la stabilité des composés, par exemple par éthérification à l'aide de dérivés de type silane par exemple ou par des groupements alkoxy en $C_1$ à $C_7$.

Il est également possible, afin d'augmenter la stabilité des composés, de remplacer une fonction hydroxy fixée directement sur le squelette stéroïdique par une chaîne hydroxylée, en particulier une chaîne hydroxy-alcoxy, de préférence en $C_1$ à $C_7$.

Lorsque des dérivés d'œstrogènes stéroïdiques sont mis en œuvre, on utilisera, de préférence, des éthers en position 3 ou 17 et/ou des dérivés hydroxyalcoxy en position 3.

Dans la limite de la définition générale, il est possible d'utiliser, dans les composés selon l'invention, un composé organométallique d'un type quelconque, en particulier des composés organométalliques de métaux des groupes VIa, VIIa ou VIII de la Classification Périodique, notamment le chrome, le molybdène, le tungstène, le manganèse, le cobalt ou le nickel, le technétium, le rhénium.

Les ligands de ces composés organométalliques peuvent être très variés, notamment CO, CS, CSe, $CNR_1$, $P(R_2,R_3,R_4)$, cyclopentadiényle, $R_1$ étant notamment un radical alkyle ou $—COR_5$ et $R_2$, $R_3$, $R_4$ et $R_5$ étant notamment des radicaux phényle ou phénoxy substitué ou non, alkyle ou alcoxy en $C_1$ à $C_7$ substitué ou non ou bien un atome d'halogène, $R_5$ pouvant être $—N(CH_2CH_2Cl)_2$.

Parmi ces composés organométalliques, il faut citer plus particulièrement les greffons de formule :

M (CO) (L') (L")

dans laquelle M est un métal du groupe VIa et L' et L", indépendamment, sont des ligands mentionnés précédemment, par exemple $Cr(CO)_3$, $Cr(CO)_2CS$, $Cr(CO)_2CSe$, $Cr(CO)_2CNCOØ$, $Cr(CO)_2PØ_3$, $Cr(CO)_2P(OØ)_3$, $Cr(CO)_2PF_3$, $Mo(CO)_3$, $W(CO)_3$ (Ø = radical phényle).

Ces composés organométalliques sont plus particulièrement destinés à être fixés sur un cycle aromatique du composé œstrogène.

Il est également possible de fixer sur les œstrogènes ou les dérivés d'œstrogènes, en particulier lorsque celui-ci comporte une triple liaison, des greffons de formule :

(M' M") (L)

dans laquelle M' et M" représentent, indépendamment, un métal du groupe VIa, VIIa ou VIII, en particulier le manganèse, le cobalt, le nickel, le molybdène, et L représente les ligands qui peuvent être choisis comme indiqué précédemment pour L' et L", par exemple $Co_2(CO)_6$, $Co(CO)_3NiCp$, $Co(CO)_3Mo(CO)_2Cp$, $Mn(CO)_4NiCp$ (Cp : cyclopentadiényle).

Parmi les composés précédents, ceux de formule :

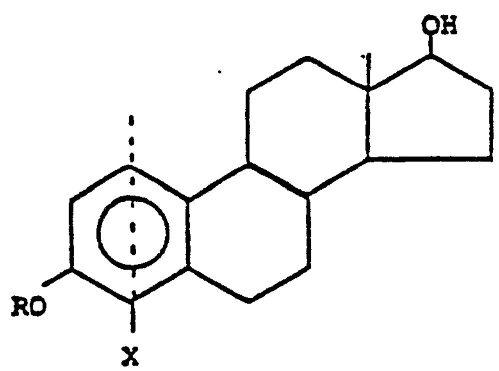

dans laquelle R est un radical protecteur ou un groupement hydroxy-alkyle et X est un composé organométallique, ce sont révélés particulièrement intéressants, notamment lorsque R est un radical silyle ou un radical $HO(CH_2)_n—$, n étant compris entre 1 et 7.

De même les composés de formule :

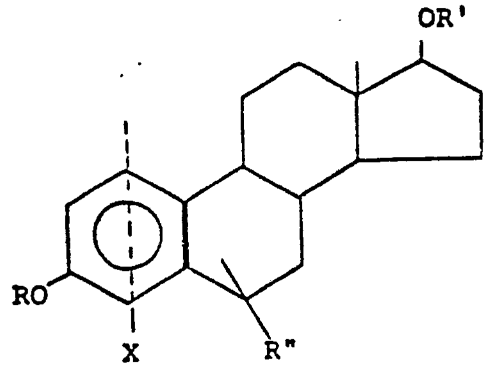

dans laquelle R et R' et X peuvent avoir les significations données précédemment et R" est de préférence un radical $HO(CH_2)_n—$, n étant compris entre 1 et 7.

Les composés selon la présente invention peuvent être préparés par des procédés connus, notamment par action sur le composé œstrogène, ou le dérivé de composé œstrogène, du dérivé organométallique correspondant. Bien entendu, lorsque cela sera nécessaire, certaines des fonctions du composé œstrogène pourront être protégées, notamment par silylation, là encore grâce à des procédés connus.

La présente invention concerne également l'application de ces composés au dosage des récepteurs

hormonaux par des techniques de spectrographies infrarouge ou Raman notamment.

Dans ce type de procédé de détection et de dosage, on met en présence les composés selon la présente invention et les échantillons porteurs de récepteurs spécifiques et on détecte par spectrographie infrarouge ou raman la présence des composés selon la présente invention fixés sur les récepteurs hormonaux par leur bande de fréquence au voisinage de 1 860 à 2 000 $cm^{-1}$.

Comme cela sera démontré dans les exemples ci-après, ce procédé permet de détecter des quantités très faibles de composés, sans faire appel à des produits radioactifs dont les inconvénients sont bien connus.

Par ailleurs, le procédé de dosage ainsi mis au point entre parfaitement dans la gamme des sensibilités requises pour les applications actuelles, à savoir notamment la détection des cancers primaires du sein.

Ce procédé présente, en outre, l'avantage de ne nécessiter qu'un temps de mesure très bref (environ 1 heure).

Les exemples ci-après sont destinés à illustrer la présente invention.

Les solvants sont désignés par : éther = E ; éther de pétrole = Ep. Les spectres de RMN ont été relevés sur l'appareil VARIAN EM 360.

Exemple 1

a) Synthèse du β-œstradiol mono-silylé

Dans un ballon purgé d'azote, on met en suspension 1,2 g ($2,5 \cdot 10^{-2}$ mole) de NaH à 50 % dans 40 ml de THF. On ajoute ensuite doucement 5,54 g ($2 \cdot 10^{-2}$ mole) d'œstradiol dissous dans 40 ml de THF. Une demi heure après, 3,5 g ($2,33 \cdot 10^{-2}$ mole) de t · $BuMe_2SiCl$ sont ajoutés à l'état solide dans le milieu réactionnel et on laisse tourner pendant 3 heures. Le mélange est ensuite versé avec précaution dans l'eau glacée, puis on extrait le produit au $CH_2Cl_2$. Après lavage et évaporation du solvant, on obtient 7,86 g de solide blanc. La recristallisation dans l'éther de pétrole donne facilement 5,7 g du composé de formule 1 ; F 158°, 73 %, cristaux blancs.

Analyse : $C_{27}H_{38}O_9CrSi$.
trouvé : C 74,95 H 9,97 ;
calculé : C 74,57 H 9,91 ;
RMN ($CDCl_3$) cycle δ = 7,2 d, 6,7 dd, 6,61 d ;
Me δ = 0,86 s ; $Me_2$ δ = 0,23 s ; t · Bu δ = 1,03 s.

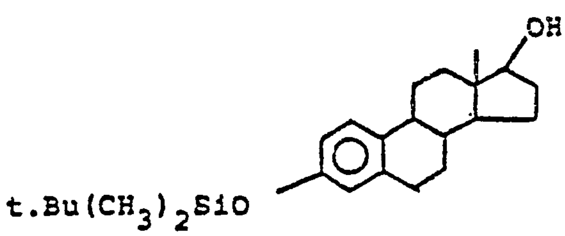

(1)

b) Complexation du β-œstradiol mono-silylé

Dans un ballon de 250 ml, 3,2 g ($8,3 \cdot 10^{-3}$ mole) de β-œstradiol mono-silylé préparé en a) et 3,7 g ($1,6 \cdot 10^{-2}$ mole) de $Cr(CO)_6$ sont mis avec 150 ml d'éther dibutylique fraîchement distillé. On chauffe à reflux, sous atmosphère d'azote, pendant 8 heures. Après évaporation de solvant, le brut réactionnel, 6,18 g, solide jaune, est chromatographié sur colonne de gel de silice 7734 avec comme éluant E/Ep = 1/1.

On isole :

— fraction de tête : produit 2, 1,27 g, 29 %, F 220° (E/Ep), cristaux jaunes.
Analyse : $C_{27}H_{38}O_5CrSi$,
trouvé : C 61,82 H 7,27
calculé : C 62,05 H 7,33
RMN ($CDCl_3$) cycle δ = 5,76 d, 5,03 dd, 4,97 d ;
Me δ = 0,73 s ; $Me_2$ δ = 0,20 s ; t · Bu δ = 0,90 s ;

— fraction de queue : produit 3, 1,8 g, 41,5 %, F 179° (E/Ep), cristaux jaunes.
Analyse : $C_{27}H_{36}O_5CrSi$,
trouvé : C 62,00 H 7,30
calculé : C 62,05 H 7,33
RMN ($CDCl_3$) cycle δ = 5,65 d, 4,96 d, 4,90 dd ;
Me δ = 0,83 s ; $Me_2$ δ = 0,26 s ; t · Bu δ = 0,96 s.

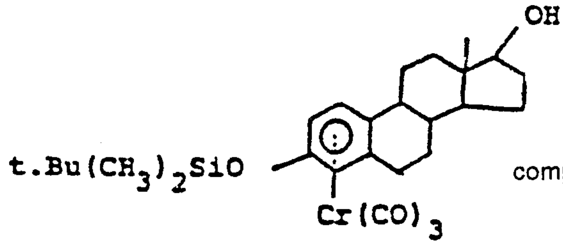

(2)

complexe α

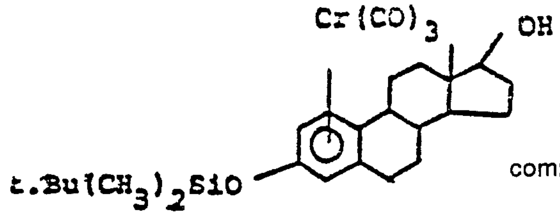

(3)

complexe β

Exemple 2

a) Complexe α du β-œstradiol disilylé

Par silylation du composé 2 avec un excès de NaH et de chlorure de silane, on obtient le complexe du titre.

Complexe œstradiol mono-silylé 2 : 770 mg ($1,47 \cdot 10^{-3}$ mole), NaH : 600 mg ($1,2 \cdot 10^{-2}$ mole, $t \cdot BuMe_2SiCl$ : 900 mg ($6 \cdot 10^{-3}$ mole).

Après extraction à l'éther et évaporation du solvant, on obtient 1,9 g de solide jaune que l'on purifie sur colonne de gel de silice 7734 avec un éluant E/Ep = 1/7.

On isole finalement 0,9 g de produit, 96 %, F 225° (E/Ep), cristaux jaunes.

Analyse : $C_{33}H_{52}CrO_5Si_2$,
trouvé : C 62,32 H 8,27
calculé : C 62,22 H 8,23
RMN ($CDCl_3$) cycle δ = 5,80 d, 5,03 dd, 4,97 d ;
Me δ = 0,73 s ; $Me_2$ δ = 0,26 s, 0,09 s ; t · Bu δ = 0,96 s, 0,90 s.

b) Complexe β du β-œstradiol disilylé

Le mode opératoire est identique à celui de son diastéréoisomère. En partant du composé 3, on obtient le produit avec 93 % de rendement, F 253° (E/Ep), cristaux jaunes.

Analyse : $C_{33}H_{52}CrO_5Si_2$,
trouvé : C 62,23 H 8,21
calculé : C 62,22 H 8,23
RMN ($CDCl_3$) cycle δ = 5,66 d, 4,94 d, 4,87 dd ;
Me δ = 0,75 s ; $Me_2$ δ = 0,21 s, — 0,05 s ;
t · Bu δ = 0,90 s, 0,83 s.

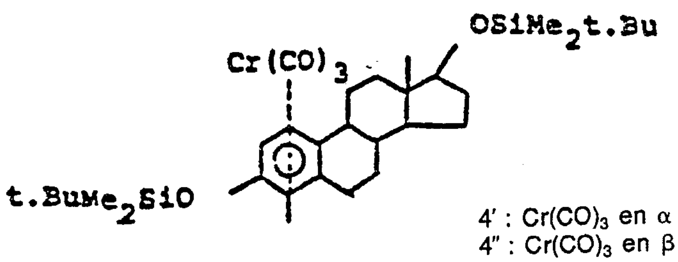

(4)

4' : $Cr(CO)_3$ en α
4" : $Cr(CO)_3$ en β

Exemple 3

Synthèse du composé 5

On irradie pendant 3 heures une solution d'œstradiol mono-silylé α chrome tricarbonyle (composé 2), 0,52 g ($10^{-3}$ mole), dans 150 ml de benzène et 25 ml de cyclooctène où barbote un courant d'azote. On ajoute ensuite 1 g de $P(Ph)_3$ et 25 ml de $CS_2$ et on chauffe à 50° pendant 1 heure 1/2. Après filtration et évaporation, le brut est chromatographié sur colonne de gel de silice Merck 9385 avec comme éluant E/Ep = 3/2. On isole finalement 60 mg du produit désiré, 11 %, F 142° (E/Ep).

RMN ($CDCl_3$) cycle δ = 5,93 d (1), 5,24 dd (1), 5,11 d (1) ;

Me δ = 0,78 s ; $Me_2$ δ = 0,25 s ; t · Bu δ = 0,93 s.

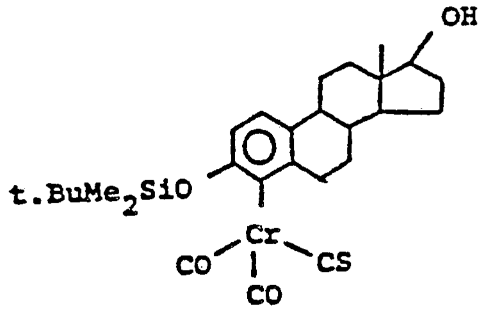

complexe α (5)

Exemple 4

a) Dérivé propanol éther de l'œstradiol

On chauffe à reflux pendant 15 heures 1,08 g d'œstradiol ($4 \cdot 10^{-3}$ mole) avec 320 mg de soude ($8 \cdot 10^{-3}$ mole) dissous dans 50 $cm^3$ d'acétone, puis 1,4 g de bromopropanol ($10^{-2}$ mole) est ensuite ajouté et le chauffage est maintenu pendant 2 jours.

Après filtration et évaporation, le résidu obtenu est redissous dans $CH_2Cl_2$. La solution obtenue est d'abord lavée à l'eau jusqu'à pH neutre, puis séchée sur $MgSO_4$, filtrée et évaporée. On a alors un solide blanc que l'on lave au pentane. On obtient finalement une masse de 1,3 g.

La chromatographie sur couche mince (éluant E/Ep = 2/1) révèle que le produit est pur (Rendement : 100 %).

La recristallisation dans l'éther donne des cristaux blancs, F 168°, 900 mg.

RMN ($CD_3COCD_3$) cycle δ = 7,31 d (1), 6,83 dd (1), 6,74 d (1) ; $CH_3$ δ = 0,83 s (3) ; $OCH_2$ δ = 4,14 t (2).

Analyse : $C_{21}H_{30}O_3$,
trouvé : C 76,05 H 9,17
calculé : C 76,32 H 9,15
Masse : $M^+/C$ = 330,2197 $[\alpha]_D^{21}$ = 70,6 ($CH_2Cl_2$, C = 0,51.

b) Complexation du dérivé propanoléther de l'œstradiol

Dans un ballon de 250 ml, on met 600 mg du composé précédent ($1,8 \cdot 10^{-3}$ mole), 1,1 g de Cr $(CO)_6$ ($5 \cdot 10^{-3}$ mole) et 150 $cm^3$ d'éther dibutylique. On chauffe à reflux le mélange pendant 6 heures. La solution devient jaune limpide.

Après évaporation, on obtient 1,24 g d'huile jaune contenant plusieurs produits.

La chromatographie sur plaques épaisses de gel de silice 7731 avec éluant THF/Ep = 2/3, permet d'isoler deux produits :

— en tête : composé 6, complexe α, 270 mg, rendement 32 %, solide jaune. La cristallisation dans E/Ep donne des cristaux jaunes, F 130°.

RMN ($CD_3COCD_3$) cycle δ = 6,08 d (1), 5,40 dd (1), 5,34 d (1) ; $CH_3$ δ = 0,67 s (3) ; $OCH_2$ δ = 4,0 t (2).
$[\alpha]_D^{21}$ = 41,7 ($CH_2Cl_2$, C = 1,08). Masse : 466 = $M^+$, 382 = $M^+$ — 3 CO, 330 = $M^+$ — $Cr(CO)_3$.
Analyse : $C_{24}H_{30}O_6Cr$,
trouvé : C 61,30 H 6,68
calculé : C 61,79 H 6,48

— en queue : composé 7, complexe β, 210 mg, rendement 25 %, solide jaune. La cristallisation dans E/Ep donne des cristaux jaunes, F 157°.

RMN ($CD_3COCD_3$) cycle δ = 6,00 d (1), 5,31 d (1), 5,23 dd (1) ; $CH_3$ δ = 0,78 s (3), $OCH_2$ δ = 4,04 t (2)
$[\alpha]_D^{21}$ = 70,0 ($CH_2Cl_2$, C = 1,08). Masse : 466 = $M^+$, 382 = $M^+$ —3 CO, 330 = $M^+$ — $Cr(CO)_3$
Analyse : $C_{24}H_{30}O_6Cr$,
trouvé : C 61,84 H 6,60
calculé : C 61,79 H 6,48

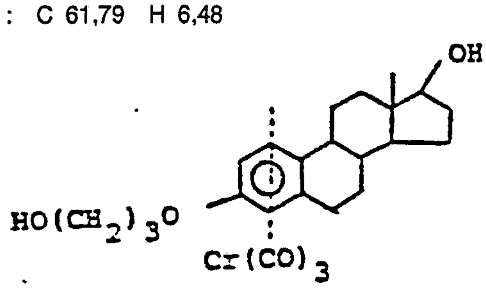

6 : $Cr(CO)_3$ en α
7 : $Cr(CO)_3$ en β

Exemple 5

(Ethynyl œstradiol) $Co_2(CO)_6$

Sous atmosphère d'argon, on ajoute doucement 0,6 g d'éthynyl œstradiol ($2 \cdot 10^{-3}$ mole) dissous dans 20 ml d'éther anhydre dans une solution de 1,05 g de $Co_2(CO)_8$ ($3 \cdot 10^{-3}$ mole) dans 10 ml d'éther. On laisse la réaction se poursuivre pendant 1 heure, puis on filtre et évapore le solvant. Le brut obtenu est purifié sur colonne de gel de silice Merck 8395 avec éluant E/Ep : 1/1. On obtient finalement 1 g de produit désiré, solide rouge sang. Il est difficile de donner un point de fusion exact à cause de la décomposition du produit.

RMN ($CDCl_3$) cycle $\delta = 6{,}63$ d (1), 6,70 dd (1), 7,23 d (1) ; 3 — OH $\delta = 6{,}21$ s (1) ; $CH_3$ $\delta = 1{,}1$ (3)
Analyse : $C_{26}H_{24}Co_2O_8$,
trouvé : C 53,80 H 4,61 Co 19,70
calculé : C 53,62 H 4,15 Co 20,24

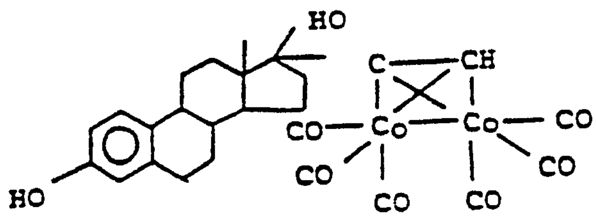

(8)

Exemple 6

Préparation du dérivé 6-hydroxyméthyl du 3-benzyloxy 17 β t · butyl-diméthylsiloxy œstradiol chrome tricarbonyle.

Le schéma ci-après résume la synthèse du composé en cause.

(Voir Schéma page 8)

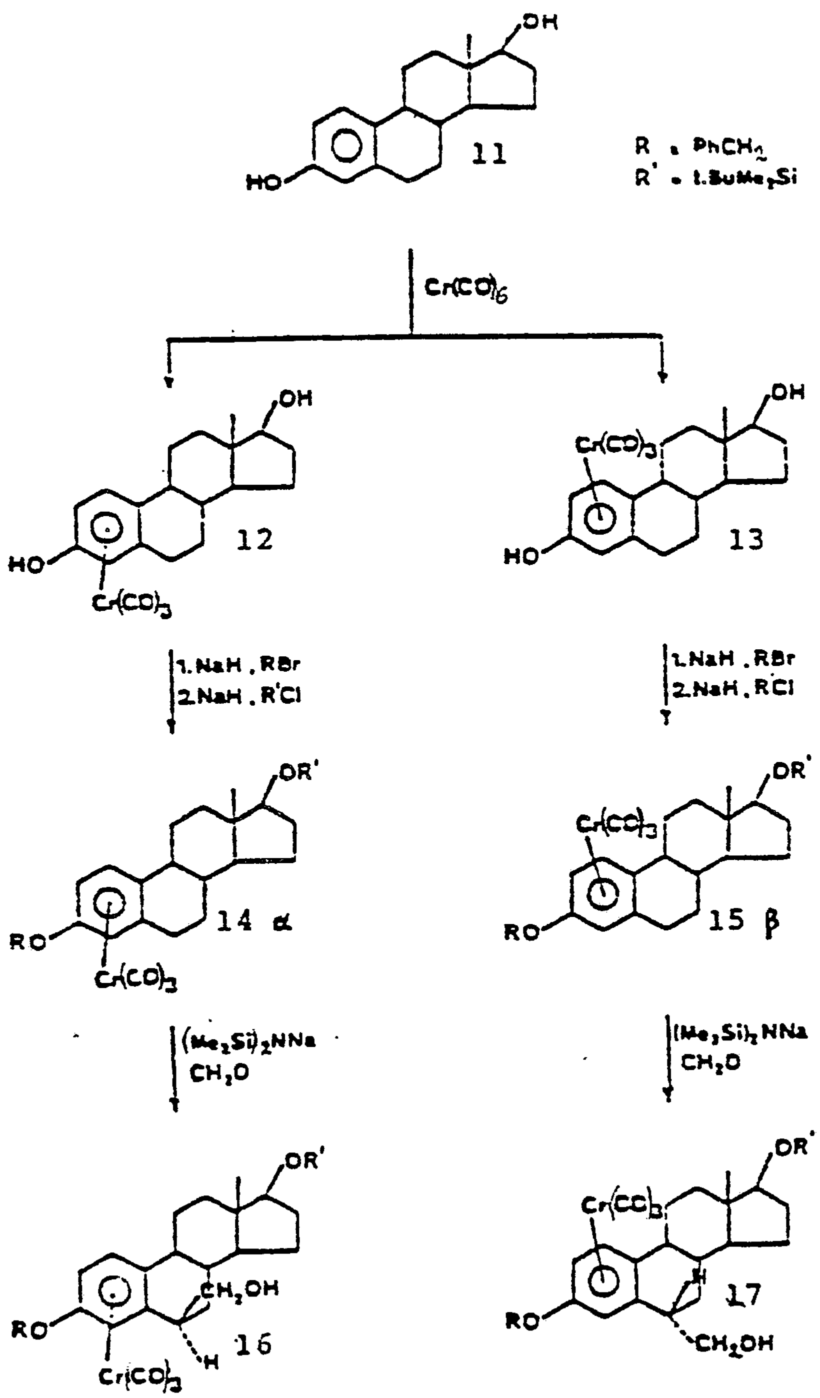
11
R = $PhCH_2$
R' = t.Bu$Me_2$Si
$Cr(CO)_6$
12
13
1. NaH, RBr
2. NaH, R'Cl
14 α
15 β
$(Me_3Si)_2NNa$
$CH_2O$
16
17

Schéma

L'œstradiol est complexé par chauffage avec $Cr(CO)_6$ dans l'éther dibutylique.

Le mélange des deux dérivés $CrCO_3$ œstradiol α et β est traité rapidement avec NaH et $C_6H_5CH_2Br$.

Les deux dérivés 3' benzyloxy sont séparés sur une colonne de silicagel (éluant : éther/éther de pétrole : 2/1). Chaque diastéréomère est traité avec NaH et t-$BuMe_2SiCl$ pour donner les produits 4 et 5 avec un rendement de 45 %. L'identification des diastéréomères est effectuée par corrélation chimique avec un composé de type correspondant dont la structure a été déterminée par analyse aux rayons X.

Les diastéréomères 14 et 15 sont mis en réaction séparément avec $(Me_3Si)_2NN_a$ et le formaldéhyde dans le OMSO.

On obtient ainsi stéréospécifiquement les complexes 16 et 17 avec un rendement de 56 %, pour le complexe 16 et avec un rendement de 62 % pour le complexe 17.

Ces complexes peuvent être des précurseurs intéressants dans l'étude et la solution des problèmes endocrinologiques, par exemple dans la fixation des groupes cytotoxiques, la préparation d'œstrogène émettant des γ et des marqueurs d'affinité.

## Exemple 7

### Mise en évidence des récepteurs hormonaux à l'aide des composés selon l'invention

Différents composés selon la présente invention ont été testés pour leur pouvoir compétiteur vis-à-vis de l'œstradiol, grâce à des courbes Log/log it, comme décrit dans Rao et al. Endocrinology, 92, 1229 (1973).

Les résultats sont rassemblés dans le tableau I ci-après :

Tableau I

| Composé | RBA (%) |
|---|---|
| Oestradiol | 100 |
| éthynyloestradiol | 71,4 |
| 1 | 11 |
| 2 | 1,05 |
| 3 | 0,36 |
| 4' | aucune compétition |
| 4" | aucune compétition |
| 5 | 1,5 |
| 6 | 28 |
| 7 | 1,2 |
| 8 | 3,5 |

Il faut rappeler que, plus la valeur de RBA (« Relative Binding Affinity » s'éloigne de 100, plus le pouvoir compétiteur du stéroïde testé est faible. Afin de fixer les idées, notons qu'un médicament aussi utilisé que le tamoxifène (Novaldex) présente un RBA de 3 %.

On constate à la lecture de ce tableau que, le marquage par une entité organo-métallique diminue le pouvoir compétiteur de l'hormone ainsi modifiée.

Cependant, les composés selon l'invention présentent, sauf pour le composé 3, une préservation du pouvoir de reconnaissance du récepteur tout à fait compatible avec une utilisation de ces produits à des fins analytiques.

Ces complexes de l'invention sont stables à l'état solide et peuvent être conservés en milieu non-oxydant sans problème.

Toutefois, la présence d'une fonction hydroxy phénolique adjacente d'un groupement organométallique est incompatible avec la stabilité du produit, qui se décompose rapidement. A ce propos, il convient de noter qu'un composé du type de ceux de l'invention a été décrit par G. Pouskouleli, I. S. Butler et J. P. Hickey, J. Inorg. Nucl. Chem., 42, 1659-1662 (1980), mais il s'agit précisément d'un composé ayant une fonction phénol adjacente du site de fixation du composé organométallique, ce qui rend ce composé instable.

Ce tableau montre que la préservation des fonctions hydroxylées en 3 ou en 17 dans ces produits est nécessaire pour obtenir un bon taux de compétitivité, à titre d'exemple, les composés 4' et 4" ne présentent aucune compétition, il est nécessaire de trouver des solutions conciliant les deux impératifs précédents.

Comme cela ressort du tableau, il est d'abord possible d'effectuer la complexation loin du groupement hydroxy phénolique, par exemple en 17α comme dans le composé 8.

Il est possible également de protéger le groupement en position 3 par éthérification, comme dans les composés 2 et 3 par exemple, tout en conservant la fonction hydroxy en 17 libre.

Enfin, il est possible en quelque sorte « d'éloigner » la fonction hydroxy libre en position 3 en greffant dans cette position un radical hydroxy-alcoxy, comme dans les produits 6 et 7.

Le résultat le plus spectaculaire est fourni par le produit 6 (RBA 28 %) dans lequel la greffe d'une

chaîne $(CH_2)_3OH$ en 3 associée à une complexation de la face α à l'aide du groupe $Cr(CO)_3$ offre une solution excellente au problème.

Exemple 8

Détection de récepteur spécifique d'œstradiol

Les échantillons utilisés dans cet exemple ont été préparés à partir de cytosols utérins de brebis partiellement purifiés par précipitation au sulfate d'ammonium à 35 %, procédé qui présente le double avantage d'atténuer l'absorbance parasite dans la région spectrale à étudier et d'éliminer la majorité des associations protéiniques non liantes avec l'hormone. (J. Katznellenbogen et al., « Cytotoxic estrogens in Hormone Receptive Tumors, Acad. Press. London (1980) p. 3-38).

Pour la préparation des échantillons destinés à l'étude I. R., on a utilisé la technique de précipitation au sulfate de protamine (A.W. Steggles et R. J. B. King, J. Biochem. 118, 695-701 (1970)) qui fournit une poudre blanche utilisable sans autre traitement.

Les figures ci-annexées permettent de comprendre les applications des composés selon l'invention.

Figure 1

Spectre I. R. des protéines présentes dans le cytosol de brebis purifiées et traitées comme précisé précédemment. Il y a lieu de noter la remarquable « fenêtre » dans la région de 1 950 $cm^{-1}$ et la faible absorbance à cet endroit. Le reste de la région spectrale se caractérise par la présence de massifs d'absorption (maxima hors échelle dans le cas présent pour plus de clarté). Le spectre comme les suivants a été obtenu à l'aide d'un appareil I. R. F.T. Nicolet 6000c, l'échantillon solide pesé et pressé est simplement porté par des « micropellets ». Si la protéine réceptrice de l'œstradiol est présente dans l'échantillon étudié seul un ligand marqué résonnant de façon intense et spécifique vers 1 900 $cm^{-1}$ en permettra la détection.

Figure 2

Spectre I. R. de l'hormone modifiée 2 en solution. On note les deux bandes intenses $\bar{\nu}$ CO de modes $A_1$ et E respectivement à 1 959,6 $cm^{-1}$ et 1 876,4 $cm^{-1}$. Ce produit, à taux de compétition moyen, a été utilisé dans la suite de l'expérience relatée ici.

Figure 3

Même échantillon que celui de la figure 1, mais traité par l'hormone 2. On note les deux pics au-dessous de 2 000 $cm^{-1}$. Cette région a été élargie dans un souci de clarté. Il faut noter qu'il y a donc bien du récepteur de l'œstradiol dans le mélange des protéines étudiées et que celui-ci est détectable par I. R. à l'aide d'hormones modifiées. En raison des concentrations en récepteur, on n'attend pas, à ce niveau, une manifestation plus intense de sa présence.

Figure 4

Même échantillon que celui de la figure 3 obtenu après soustraction du « blanc » (figure 1). Le résultat est suffisamment spectaculaire (comparer avec l'hormone en solution : figure 2) pour se passer de commentaire.

Il faut ajouter que l'on a détecté ici 51 fentomoles de récepteur par mg de protéine, ce qui représente un taux tout à fait usuel en cancérologie. Au-dessous de 10 fm/mg de protéine le dosage est considéré comme $ER^-$ (seuil de prise en considération). Tandis que des taux dépassant 100 fm/mg de protéine sont fréquents. On est donc avec cette technique dans la gamme des sensibilités requises pour ces analyses fines.

Pour fixer les idées, notons que l'on a décelé ici $10^{-10}$ g de métal sans problème (la limite n'a pas été précisée) alors que par absorption atomique on n'arrive pas en dessous de $10^{-6}$ g de métal avec des précautions draconiennes. De plus, la morphologie typique de la courbe (caractéristique d'une symétrie $C_{3v}$ ; le léger « splitting » est dû au spectre du solide) constitue une mesure de protection contre les artéfacts éventuels. Enfin la présence de chrome dans l'échantillon a été confirmée grâce à des expériences indépendantes d'activation de neutron.

**Revendications**

1. Complexe d'œstrogène constitué d'un œstrogène ou d'un dérivé d'œstrogène complexé par un composé organométallique comportant au moins un ligand carbonyle libre, caractérisé en ce que ledit complexe d'œstrogène comporte au moins un radical hydroxy libre et aucun radical hydroxy phénolique

libre en α du site de fixation du composé organométallique, et en ce que les composés organométalliques sont des composés des métaux des groupes VIa, VIIa et VIII de la Classification Périodique.

2. Complexe d'œstrogène selon la revendication 1, caractérisé en ce que l'œstrogène est choisi parmi l'œstradiol, l'œstrone, le 16α-hydroxyestrone, l'œstriol, l'éthynylœstradiol, le diéthylstilbœstrol, l'hexœstrol, ainsi que leurs dérivés.

3. Complexe d'œstrogène selon la revendication 2, caractérisé en ce que les dérivés des œstrogènes stéroïdiques sont des éthers en position 3 ou 17 et/ou des dérivés hydroxyalcoxy en position 3.

4. Complexe d'œstrogène selon la revendication 1, caractérisé en ce que les composés organométalliques sont des composés d'un ou plusieurs métaux choisis parmi le chrome, le molybdène, le tungstène, le manganèse, le cobalt et le nickel, le technétium et le rhénium.

5. Complexe d'œstrogène selon l'une des revendications 1 à 4, caractérisé en ce que les ligands des métaux des composés organométalliques sont choisis parmi: CO, CS, CSe, $CNR_1$, $P(R_2,R_3,R_4)$, cyclopentadiényl, $R_1$ étant un radical alkyle ou $—COR_5$, $R_2$, $R_3$, $R_4$ et $R_5$ étant choisis parmi les radicaux phényle ou phénoxy substitué ou non substitué et les radicaux alkyle ou alcoxy substitué ou non substitué et les atomes d'halogène, $R_5$ pouvant être $—N(CH_2CH_2Cl_2)_2$.

6. Complexe d'œstrogène selon la revendication 5, caractérisé en ce que les composés organométalliques sont choisis parmi : $Cr(CO)_3$, $Cr(CO)_2CS$, $Cr(CO)_2CSe$, et $Co_2(CO)_6$.

7. Complexe d'œstrogène selon l'une des revendications 1 à 6, de formule :

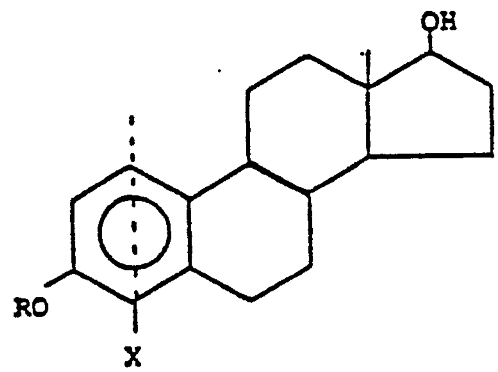

dans laquelle R est un radical protecteur ou un groupement hydroxyalkyle et X est un greffon organométallique.

8. Complexe d'œstrogène selon l'une des revendications 1 à 6 de formule

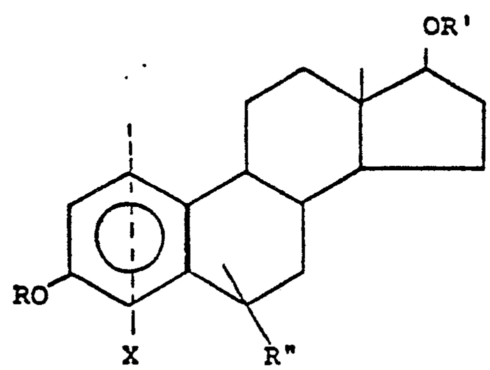

dans laquelle R et R' sont des radicaux protecteurs ou un groupement hydroxyalkyle et X est un greffon organométallique et R" est un radical hydroxylé.

9. Complexe d'œstrogène selon l'une des revendications 7 et 8, caractérisé en ce que X est le radical $Cr(CO)_3$, $Cr(CO)_2CS$, $Cr(CO)_2CSe$ et R est le radical $HO(CH^2)^n—$, n étant compris entre 1 et 7.

10. Application des composés selon l'une des revendications 1 à 9 au dosage des récepteurs hormonaux par spectrographie.

11. Application selon la revendication 10, caractérisée en ce qu'on utilise la spectrographie infrarouge ou Raman.

**Claims**

1. An estrogen complex which consists of an estrogen or an estrogen derivative complexed with an organometallic compound containing at least one free carbonyl ligand, characterized in that the said estrogen complex contains at least one free hydroxyl radical and no free phenolic hydroxyl radical in the position to the site where the organometallic compound is attached, and in that the organometallic compounds are compounds of the metals of groups VIa, VIIa, VIII of the Periodic Table.

2. An estrogen complex as claimed in claim 1, wherein the estrogen is chosen from amongst estradiol, estrone, 16-hydroxyestrone, estriol, éthynylestradiol, diethylstilbestrol, hexestrol and also derivatives thereof.

3. An estrogen complex as claimed in claim 2, wherein the derivatives of the steroidal estrogens are ethers in the 3-position or 17-position and/or hydroxy-alkoxy derivatives in the 3-position.

4. An estrogen complex as claimed in claim 1, wherein the organometallic compounds are compounds of one or more metals chosen from amongst chromium, molybdenum, tungsten, manganese, cobalt and nickel, technetium and rhenium.

5. An estrogen complex as claimed in any of claims 1 and 4, wherein the ligands of the metals of the organometallic compounds are chosen from amongst : CO, CS, CSe, $CNR_1$, $P(R_2,R_3,R_4)$ and cyclopentadienyl, $R_1$ being an alkyl radical or $-COR_5$ and $R_2$, $R_3$, $R_4$ and $R_5$ being chosen from amongst substituted or unsubstituted phenyl or phenoxy radicals and substituted or unsubstituted alkyl or alkoxy radicals and halogen atoms, it being possible for $R_5$ to be $-N(CH_2CH_2Cl)_2$.

6. An estrogen complex as claimed in claim 5, wherein the organometallic compounds are chosen from amongst : $Cr(CO)_3$, $Cr)CO)_2CS$, $Cr(CO)_2CSe$ and $Co_2(CO)_6$.

7. An estrogen complex as claimed in any of claims 1 to 6, of the formula :

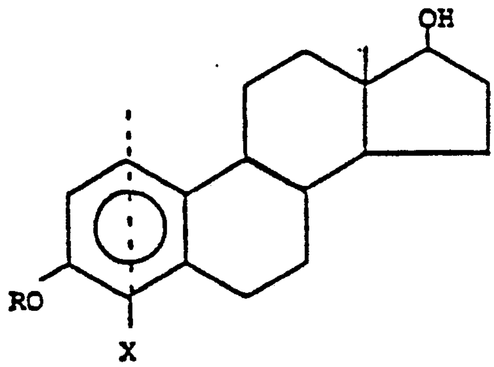

in which R is a protecting radical or a hydroxyalkyl group and X is an organometallic graft.

8. An estrogen complex as claimed in any of claims 1 to 6, of the formula :

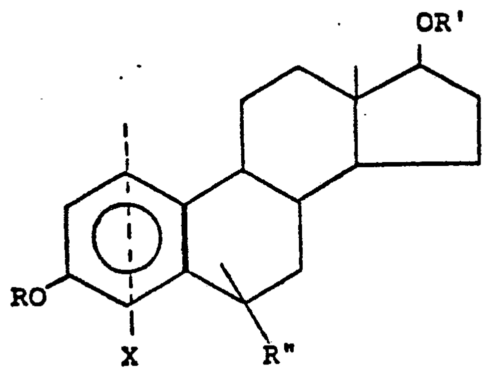

wherein R and R' are protecting group or hydroxyalkyl group, X is an organometallic graft and R" is an hydroxyalkyl.

9. An estrogen complex as claimed in claim 7 or 8, wherein X is the radical $Cr(CO)_3$, $Cr(CO)_2CS$ or $Cr(CO)_2CSe$ and R is the radical $HO(CH_2)_n-$, n being between 1 and 7.

10. Application of a compound as claimed in any of claims 1 to 9 to the determination of hormone receptors by spectrography.

11. Application as claimed in claim 10, wherein infrared or Raman spectrography is used.

**Patentansprüche**

1. Östrogenkomplex, der aus einem Östrogen oder einem Östrogenderivat besteht, das mit einer metallorganischen Verbindung komplexiert ist, die wenigstens einen freien Karbonylliganden aufweist, dadurch gekennzeichnet, daß der Östrogenkomplex wenigstens einen freien Hydroxylrest und keinen freien phenolischen Hydroxylrest in α-Position zur Bindungsstelle der metallorganischen Verbindung aufweist, und daß die metallorganischen Verbindungen Verbindungen der Metalle der Gruppen VIa, VIIa und VIII des Periodensystems sind.

2. Östrogenkomplex nach Anspruch 1, dadurch gekennzeichnet, daß das Östrogen aus Östradiol, Östron, 16α-Hydroxyöstron, Östriol, Ethinylöstradiol, Diethylstilböstrol, Hexöstrol sowie deren Derivaten ausgewählt ist.

3. Östrogenkomplex nach Anspruch 2, dadurch gekennzeichnet, daß die Derivate der Steroidöstrogene Ether in Position 3 oder 17 und/oder Hydroxyalkoxyderivate in Position 3 sind.

4. Östrogenkomplex nach Anspruch 1, dadurch gekennzeichnet, daß die metallorganischen Verbindungen Verbindungen eines oder mehrerer Metalle sind, die aus Chrom, Molybdän, Wolfram, Mangan, Kobalt und Nickel, Technetium und Rhenium ausgewählt sind.

5. Östrogenkomplex nach einem der Ansprüche 1 bis 4, dadurch gekennzeichnet, daß die Liganden der Metalle der metallorganischen Verbindungen aus CO, CS, CSe, $CNR_1$, $P(R_2,R_3,R_4)$, und Cyclopentadienyl ausgewählt sind, wobei $R_1$ ein Alkylrest oder $—COR_5$ ist und $R_2$, $R_3$, $R_4$ und $R_5$ aus substituierten oder nicht-substituierten Phenyl- oder Phenoxyresten und substituierten oder nicht-substituierten Alkyl- oder Alkoxyresten und Halogenatomen ausgewählt sind, wobei $R_5$ $—N(CH_2CH_2Cl)_2$ sein kann.

6. Östrogenkomplex nach Anspruch 5, dadurch gekennzeichnet, daß die metallorganischen Verbindungen aus $Cr(CO)_3$, $Cr(CO)_2CS$, $Cr(CO)_2CSe$ und $Co_2(CO)_6$ ausgewählt sind.

7. Östrogenkomplex nach einem der Ansprüche 1 bis 6 mit der Formel :

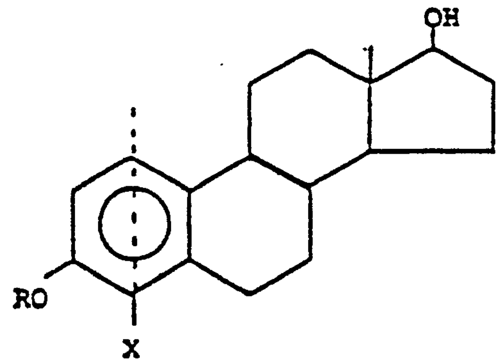

in der R ein Schutzrest oder eine Hydroxyalkyl gruppe und X ein metallorganischer Pfropf ist.

8. Östrogenkomplex nach einem der Ansprüche 1 bis 6 mit der Formel :

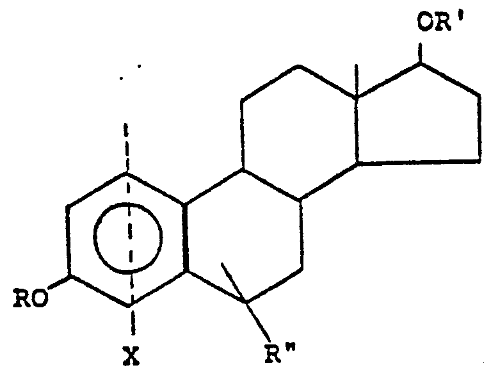

in der R und R' Schutzreste oder Hydroxyalkyl gruppen sind und X ein metallorganischer Pfropf und R" ein hydroxylierter Rest ist.

9. Östrogenkomplex nach einem der Ansprüche 7 und 8, dadurch gekennzeichnet, daß X der Rest $Cr(CO)_3$, $Cr(CO)_2CS$, $Cr(CO)_2CSe$ und R der Rest $HO(CH_2)_n—$ ist, wobei n zwischen 1 und 7 liegt.

10. Verwendung der Verbindungen nach einem der Ansprüche 1 bis 9 zur quantitativen Analyse von Hormonrezeptoren mit Spektrographie.

11. Verwendung nach Anspruch 10, dadurch gekennzeichnet, daß man Infrarot- oder Raman-Spektrographie verwendet.

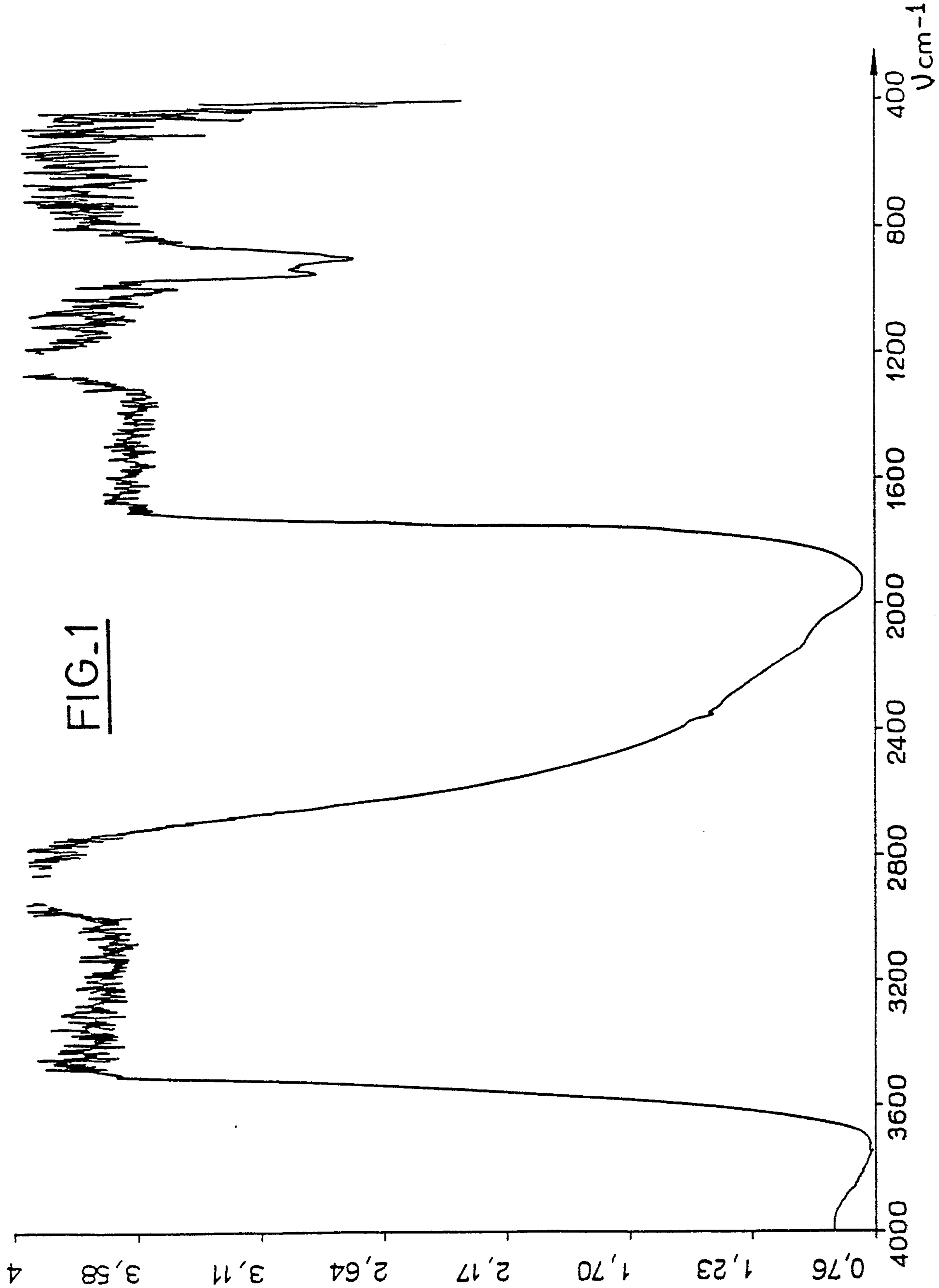
ABSORBANCE
0,76
1,23
1,70
2,17
2,64
3,11
3,58
4
4000
3600
3200
2800
2400
2000
1600
1200
800
400
Vcm-1

FIG_1

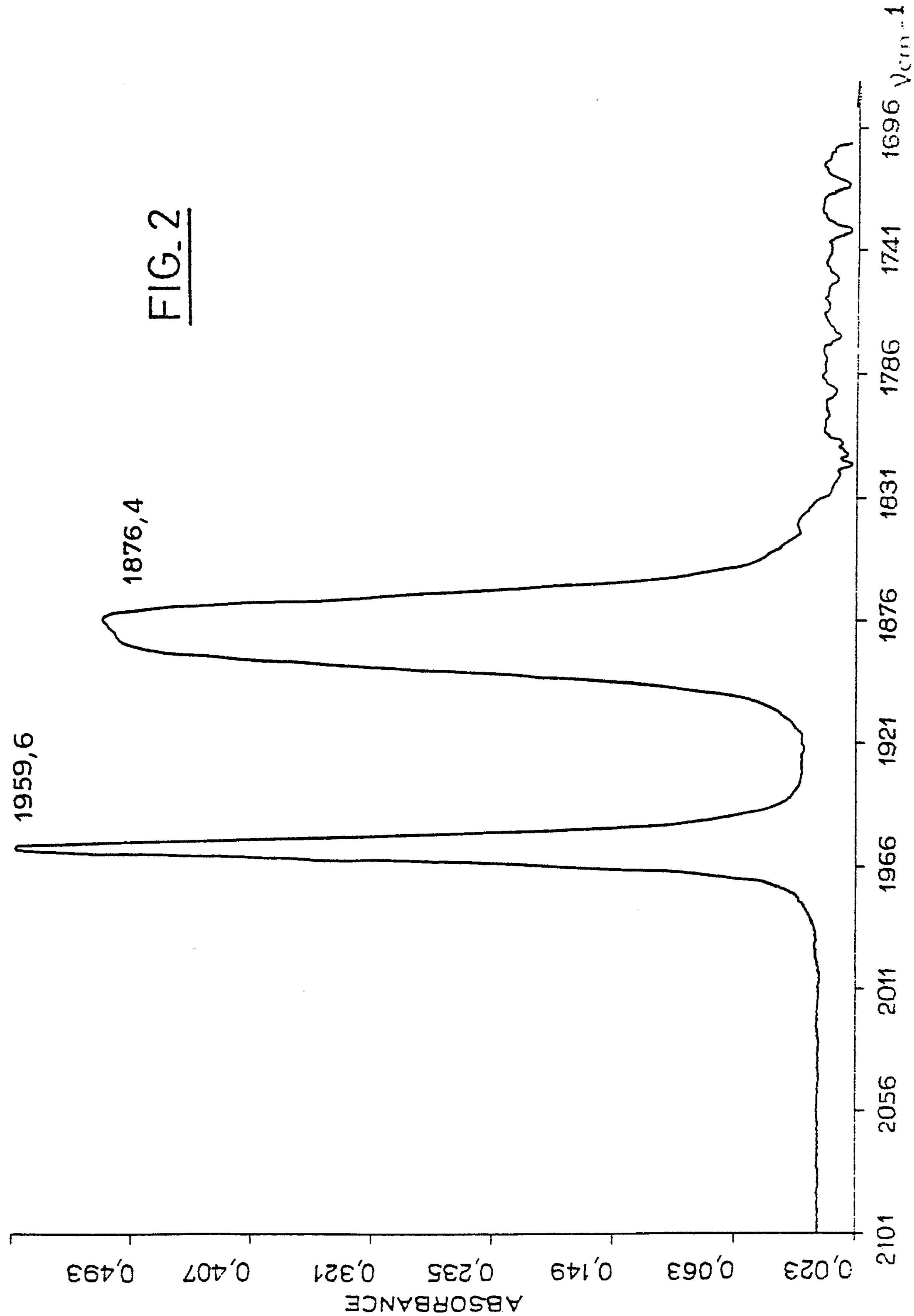
1959,6
1876,4
ABSORBANCE
0,023
0,063
0,149
0,235
0,321
0,407
0,493
2101
2056
2011
1966
1921
1876
1831
1786
1741
1696

FIG. 2

FIG. 3

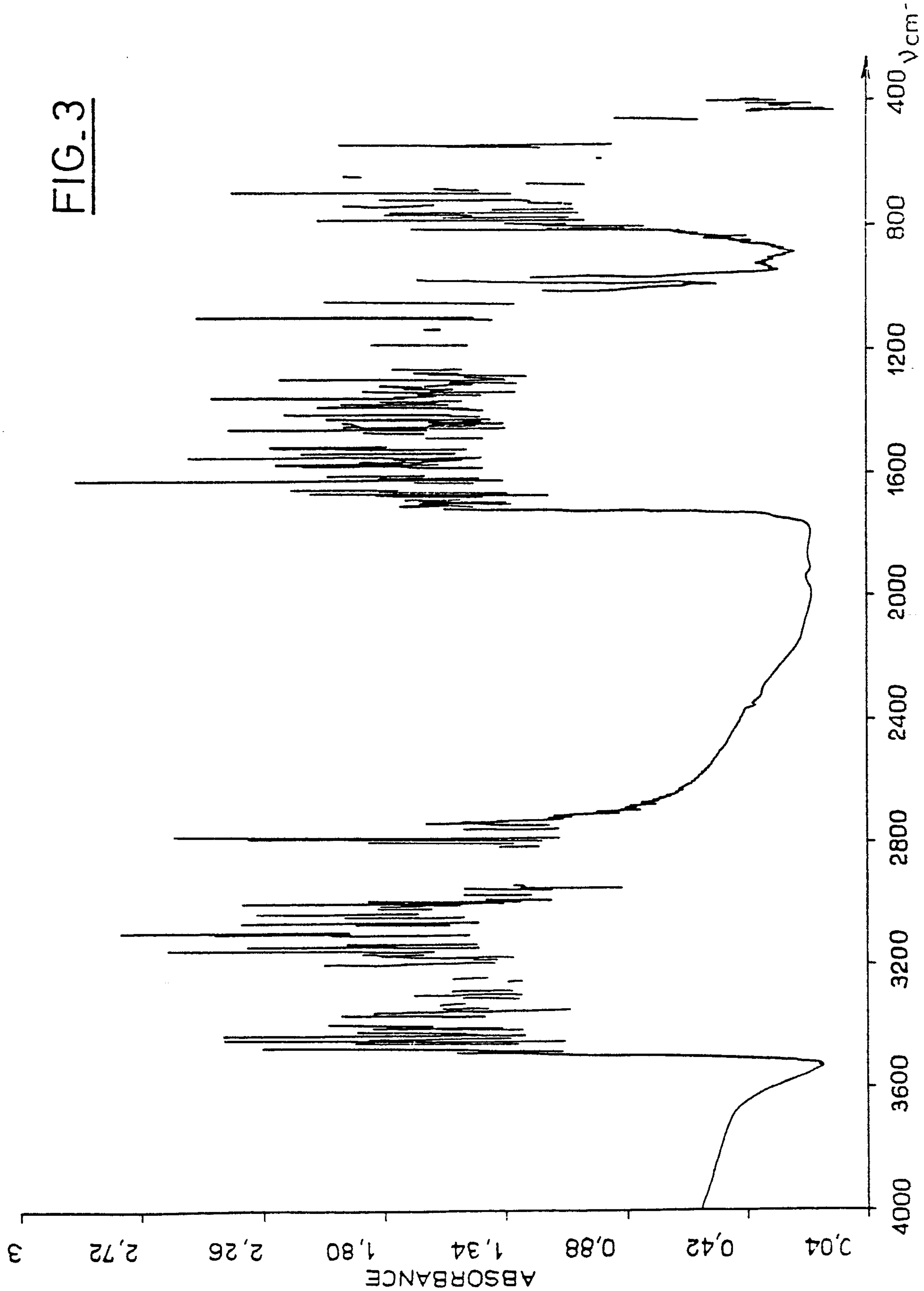
ABSORBANCE
0,04
0,42
0,88
1,34
1,80
2,26
2,72
3
4000
3600
3200
2800
2400
2000
1600
1200
800
400
ν cm-1

FIG_4

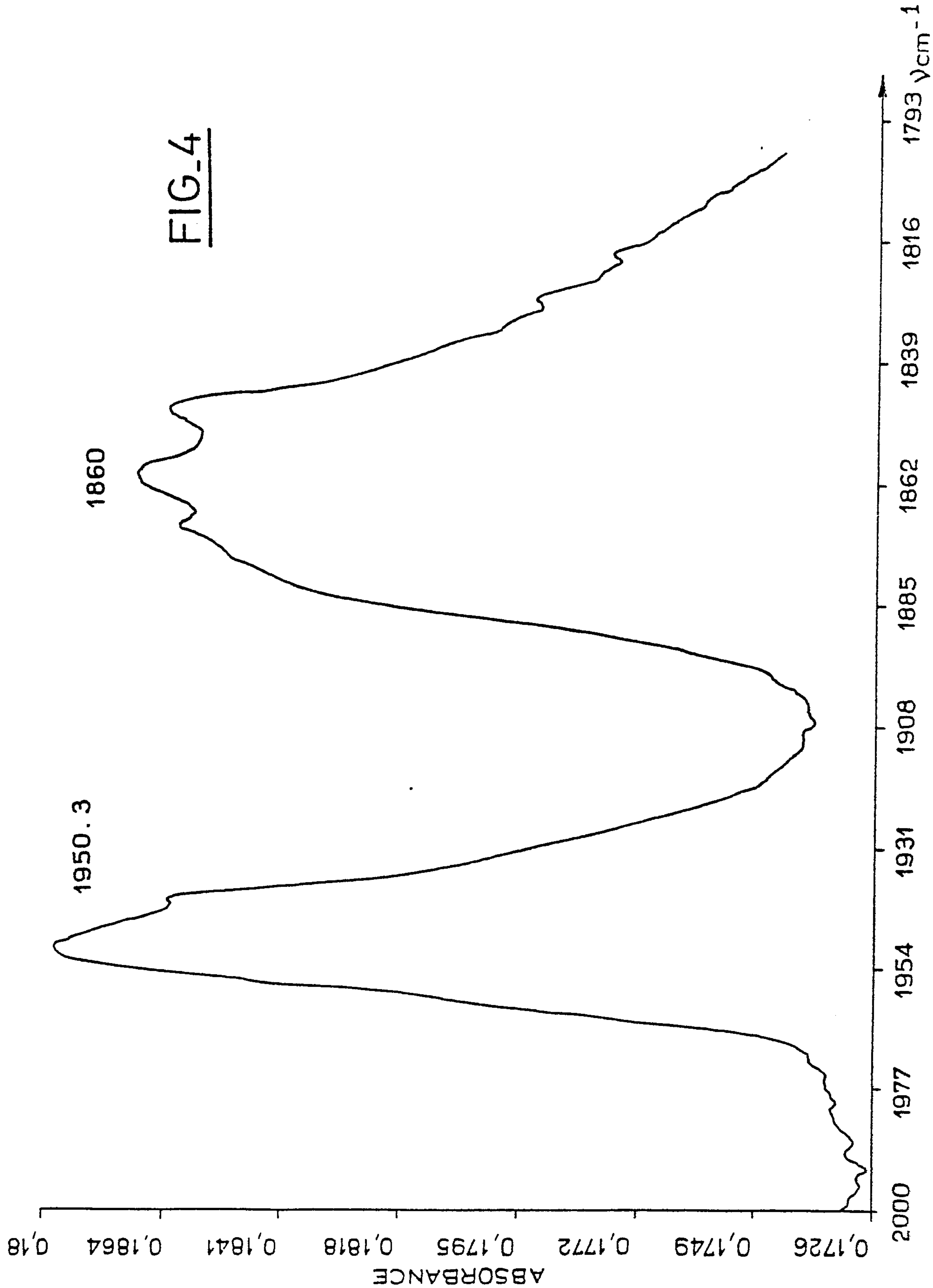

1950.3
1860
ABSORBANCE
0,18
0,1864
0,1841
0,1818
0,1795
0,1772
0,1749
0,1726
2000
1977
1954
1931
1908
1885
1862
1839
1816
1793
νcm-1